# EUROPEAN PATENT APPLICATION

(11) **EP 4 086 838 A1**
(43) Date of publication of application: **09.11.2022**
(21) Application number: 20911177.2
(22) Date of filing: 24.12.2020
(51) Int. Cl.: G06Q 50/10, G06Q 50/22, G16H 10/60

(54) **SYSTEM FOR PROVIDING GENETIC INFORMATION-BASED PERSONALIZED SOCIAL CONTENT INFORMATION, AND METHOD THEREOF**

(30) Priority: 31.12.2019 KR 20190179128
(71) Applicant: Clinomics Inc., Ulsan 44919 (KR)
(72) Inventor: CHO, Yun Sung, Yongin-si Gyeonggi-do 16953 (KR); PARK, Ye Shin, Suwon-si Gyeonggi-do 16225 (KR); CHO, Su An, Hwaseong-si Gyeonggi-do 18320 (KR); BHAK, Jong Hwa, Ulju-gun Ulsan 44936 (KR)
(74) Representative: Frenkel, Matthias Alexander
(86) International application number: PCT/KR2020/019146
(87) International publication number: WO 2021/137539

(57) **Abstract**

The present invention relates to a system for providing genetic information-based personalized social content information, and a method thereof. Disclosed, according to one embodiment, is a system for providing genetic information-based personalized social content information, the system comprising: a genetic testing device unit which performs genetic testing for genetic physical characteristics and genetic disorders of a user; a solution management item provision unit which is installed in a management server, receives a genetic testing result as input from the genetic testing device unit, and, according to the inputted genetic testing result, provides solution management items including improvement methods for and worsening factors of the genetic physical characteristics and genetic disorders of the user; a solution performance checking unit which is installed in a communication terminal of the user, receives, from the user, inputs on whether each of the solution management items, provided by the solution management item provision unit, have been performed, and transmits same to the outside at every predetermined time period; and a user management state analysis and management unit which is installed in the management server, and analyzes and manages the degree of improvement and worsening of the genetic physical characteristics and genetic disorders of the user, according to results on whether the solution management items have been performed, which are received from the solution performance checking unit.

## Description

### TECHNICAL FIELD

An embodiment of the present invention relates to a genetic information personalized social content information providing system.

### BACKGROUND ART

Current genetic testing for genetic physical characteristics or genetic diseases may test only genetic physical characteristics or disease types of individuals. There is no system to manage disease mitigation or healthcare related to current social issues, such as fine dust, and the test results.

Indeed, changes in genetic physical characteristics or diseases are affected by environmental factors as well as genetic factors.

Accordingly, a need exists for continuous management by a system for regularly providing information for enhancing healthcare associated with social issues updated in real-time and genetic test results.

[PRIOR TECHNICAL DOCUMENTS]

Korean Patent Application Publication No. 10-2014-0119022 (published on October 08, 2014)

Korean Patent Application Publication No. 10-2019-0079157 (published on July 05, 2019)

### DETAILED DESCRIPTION OF THE INVENTION

### TECHNICAL PROBLEMS

An embodiment of the present invention provides a genetic information personalized social content information providing system and method, capable of regularly providing information for enhancing healthcare associated with social issues updated in real-time and genetic test results and providing healthcare information associated with an epigenetic genetic test promptly and regularly or whenever an issue occurs.

### MEANS TO ADDRSS THE PROBLEMS

According to an embodiment of the present invention, a genetic information personalized social content information providing system comprises a genetic test device unit performing a genetic test and analysis based on a sample collected from a user to provide genetic test result information; an information collection/storage management unit collecting social issue information through an Internet communication network, extracting social issue-associated genetic test result information associated with the social issue information from the genetic test result information and storing the extracted information, interworking with the user's communication device to collect, store, and manage the user's Internet search and activity record information; and a personal genetic result and customized information providing unit providing each of the social issue-associated genetic test result information and personalized solution information related to the social issue information and according to the genetic test result information, according to at least one of a subscription service cycle selected by the user and an update time of the social issue information, wherein the personal genetic result and customized information providing unit extracts social issue and personal interest-associated genetic information based on the user's personal interest based on the search and activity record information from the social issue-associated genetic test result information and additionally provides the extracted information according to the subscription service cycle.

Further, the genetic test device unit may perform an acquired genetic test and analysis based on the sample collected from the user to add acquired epigenetic information related to a genetic marker and phenotype to the genetic test result information.

Further, the genetic test device unit may predict and analyze the user's risk of occurrence of disease and non-disease phenotype using a genetic marker to provide the genetic test result information and, when updated with a new research result according to a new genetic marker, update and provide the genetic test result information by applying the new genetic marker.

Further, the information collection/storage management unit may include a social issue information collection unit selecting and collecting the social issue information associated with pre-stored genetic marker and phenotype information through the Internet communication network; a genetic test result information selection/storage unit extracting the social issue-associated genetic test result information associated with the social issue information collected through the social issue information collection unit from the genetic test result information and storing the extracted information; and a user search and activity record information storage unit interworking with the user's communication device to collect, store, and manage Internet search and activity record information including at least one of the user's Internet search record, video view record, online shopping record, and messenger conversation.

Further, the personal genetic result and customized information providing unit may include a first information providing unit providing the user with the social issue-associated genetic test result information and the personalized solution information related to the social issue information and according to the genetic test result information, in a form of a periodical, according to the subscription service cycle; a second information providing unit providing the user with the social issue-associated genetic test result information and the personalized solution information related to the social issue information and according to the genetic test result information, in a form of a push notification popup, each time when the social issue information is updated; and a third information providing unit extracting the social issue and personal interest-associated genetic information based on the user's personal interest based on the search and activity record information from the social issue-associated genetic test result information and providing the user with the extracted information according to the subscription service cycle.

According to another embodiment of the present invention, a genetic information personalized social content information providing method comprises a genetic test step performing a genetic test and analysis based on a sample collected from a user to provide genetic test result information, by a genetic test device unit; an information collection/storage management step collecting social issue information through an Internet communication network, extracting social issue-associated genetic test result information associated with the social issue information from the genetic test result information and storing the extracted information, interworking with the user's communication device to collect, store, and manage the user's Internet search and activity record information, by an information collection/storage management unit; and a personal genetic result and customized information providing step providing each of the social issue-associated genetic test result information and personalized solution information related to the social issue information and according to the genetic test result information, according to at least one of a subscription service cycle selected by the user and an update time of the social issue information, by a personal genetic result and customized information providing unit, wherein the personal genetic result and customized information providing step extracts social issue and personal interest-associated genetic information based on the user's personal interest based on the search and activity record information from the social issue-associated genetic test result information and additionally provides the extracted information according to the subscription service cycle.

Further, the genetic test device step may perform an acquired genetic test and analysis based on the sample collected from the user to add acquired epigenetic information related to a genetic marker and phenotype to the genetic test result information.

Further, the genetic test device step may predict and analyze the user's risk of occurrence of disease and non-disease phenotype using a genetic marker to provide the genetic test result information and, when updated with a new research result according to a new genetic marker, update and provide the genetic test result information by applying the new genetic marker.

Further, the information collection/storage management step may include a social issue information collection step selecting and collecting the social issue information associated with pre-stored genetic marker and phenotype information through the Internet communication network; a genetic test result information selection/storage step extracting the social issue-associated genetic test result information associated with the social issue information collected through the social issue information collection step from the genetic test result information and storing the extracted information; and a user search and activity record information storage step interworking with the user's communication device to collect, store, and manage Internet search and activity record information including at least one of the user's Internet search record, video view record, online shopping record, and messenger conversation.

Further, the personal genetic result and customized information providing step may include a first information providing step providing the user with the social issue-associated genetic test result information and the personalized solution information related to the social issue information and according to the genetic test result information, in a form of a periodical, according to the subscription service cycle; a second information providing step providing the user with the social issue-associated genetic test result information and the personalized solution information related to the social issue information and according to the genetic test result information, in a form of a push notification popup, each time when the social issue information is updated; and a third information providing step extracting the social issue and personal interest-associated genetic information based on the user's personal interest based on the search and activity record information from the social issue-associated genetic test result information and providing the user with the extracted information according to the subscription service cycle.

### EFFECTS OF THE INVENTION

According to the present invention, there may be provided a genetic information personalized social content information providing system and method, capable of regularly providing information for enhancing healthcare associated with social issues updated in real-time and genetic test results and providing healthcare information associated with an epigenetic genetic test promptly and regularly or whenever an issue occurs.

### BRIEF DESCRIPTION OF THE DRAWINGS

FIG. 1 is a schematic diagram illustrating an overall configuration of a genetic information personalized social content information providing system according to an embodiment of the present invention.
FIG. 2 is a block diagram illustrating a configuration of a genetic information personalized social content information providing system according to an embodiment of the present invention.
FIG. 3 is a block diagram illustrating a configuration of an information collection/storage management unit according to an embodiment of the present invention.
FIG. 4 is a block diagram illustrating a personal genetic result and customized information providing unit according to an embodiment of the present invention.
FIG. 5 is a flowchart illustrating a configuration of a genetic information personalized social content information providing method according to another embodiment of the present invention.
FIG. 6 is a flowchart illustrating a configuration of an information collection/storage management step according to another embodiment of the present invention.
FIG. 7 is a flowchart illustrating a personal genetic result and customized information providing step according to another embodiment of the present invention.

### MODE TO PRACTICE THE INVENTION

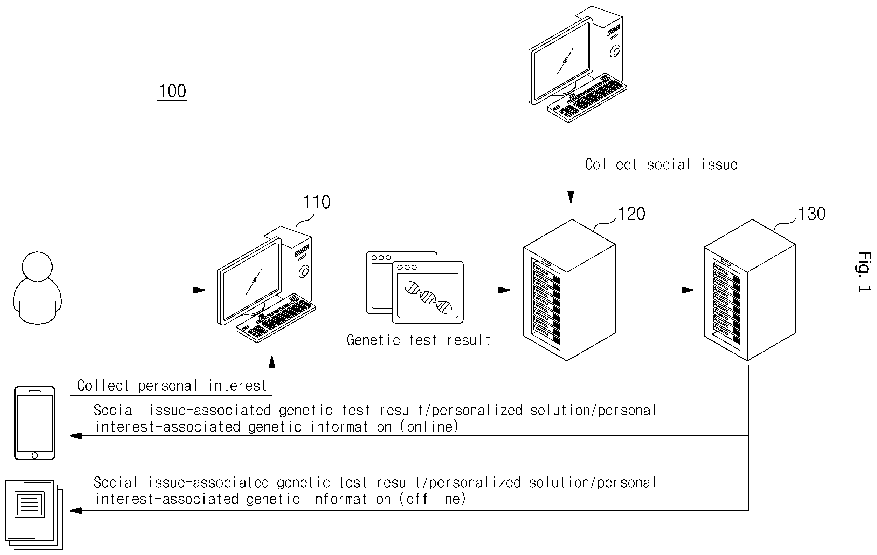

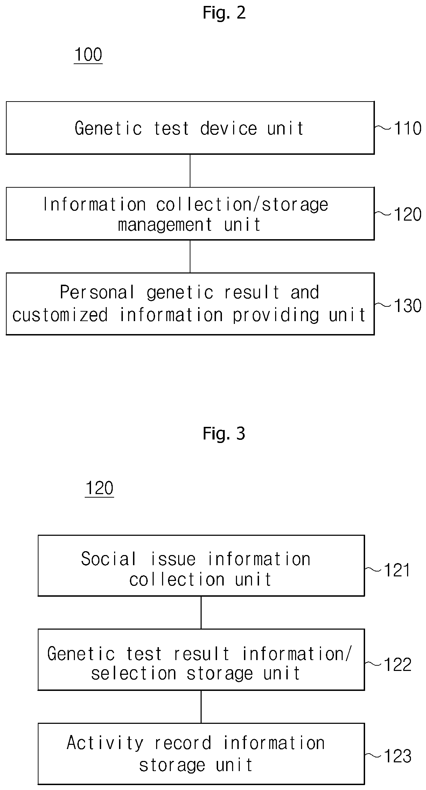

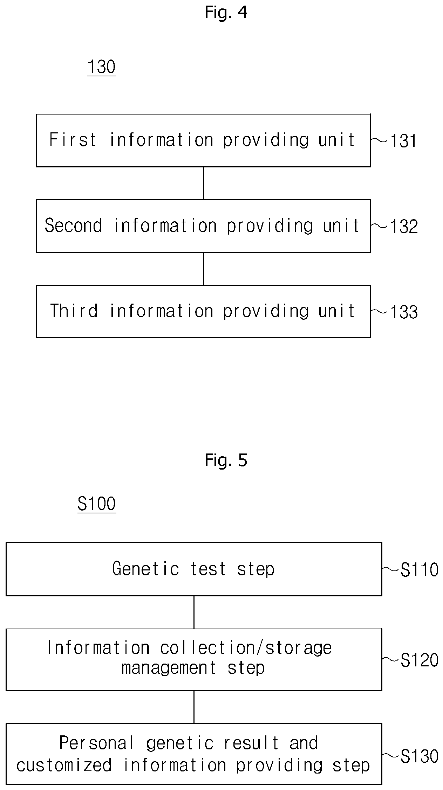

FIG. 1 is a schematic diagram illustrating an overall configuration of a genetic information personalized social content information providing system according to an embodiment of the present invention. FIG. 2 is a block diagram illustrating a configuration of a genetic information personalized social content information providing system according to an embodiment of the present invention. FIG. 3 is a block diagram illustrating a configuration of an information collection/storage management unit according to an embodiment of the present invention. FIG. 4 is a block diagram illustrating a personal genetic result and customized information providing unit according to an embodiment of the present invention.

Referring to FIGS. 1 and 2, according to an embodiment of the present invention, a genetic information personalized social content information providing system 100 includes a genetic test device unit 110, an information collection/storage management unit 120, and a personal genetic result and customized information providing unit 130.

The genetic test device unit 110 may conduct and analyze a genetic test based on a sample collected from the user and provide genetic test result information. For the genetic test, a sample, such as the user's saliva and oral epithelial cells, may be collected, and various genetic tests for genetic mutations, diseases, phenotypes, etc. may be performed on the collected sample, and the test results may be provided.

The genetic test result information (or genome analysis result data) according to the embodiment may be separated (anonymized) from personal identification information, encrypted with a separate unique number, and stored, and be protected through a server security device to prevent the data from leakage. When the user cancels the genome magazine subscription service according to the embodiment or applies for information withdrawal, the customer's genomic data and its derivative information all may be automatically deleted not to be recovered.

Further, the genetic test device unit 110 may perform and analyze an acquired genetic test based on the sample collected from the user and additionally include epigenetic information related to the genetic marker and phenotype in the genetic test result information and provide it.

More specifically, it is possible to provide more accurate genetic test results by linking and adding epigenetic information, which is acquired genetic information, with the genetic marker and phenotype according to the user's selection. In general, innate genetic information means such information as methyl group (ch3), but since such a type has the same mechanism as all other epigenetic types, such as acetyl group. Thus, fusion of innate genetic information and acquired epigenetic information is not difficult. Further, it is meaningful to provide more accurate information about the user's genes and phenotypic traits using common-sense and universal algorithms.

Further, even when the user's innate genetic information is specified, the acquired epigenetic genetic information may be enhanced through management of lifestyle, aging, and disease and may thus provide the effect of enhancing the quality of life in an acquired manner.

The genetic test device unit 110 may provide genetic test result information by predicting and analyzing the risk of occurrence of the user's disease and non-disease phenotypes by using the genetic marker, but when updated with a new research result according to a new genetic marker, update the genetic test result information to which the new genetic marker has been applied and provide it. When the existing genetic test result item is updated with the new research result, the genetic test device unit 110 adds the corresponding genetic marker information to its own database and collects and stores it.

As such, when the existing genetic test result item is updated with a new research result, the test result update providing method according to the new research result by the genetic test device unit 110 may apply the new research result thereto to additionally provide a more accurate test result. The genetic test result analysis may be performed in a method of predicting the likelihood (risk) of the occurrence of disease and non-disease phenotypes of the testee by way of the genetic markers reported through existing research. However, the results of genetic research are continuously being published. When a new genetic marker is discovered and new research results are applied, the test results (risk level) may change, so that the test results need to be updated accordingly.

The information collection/storage management unit 120 may collect social issue information through an Internet communication network, extract and store social issue-associated genetic test result information, associated with the social issue information, among the genetic test result information and interwork with the user's communication device to collect and store the user's Internet search and activity record information.

To this end, the information collection/storage management unit 120 may include a social issue information collection unit 121, a genetic test result information/selection storage unit 122, and a user search and activity record information storage unit 123 as shown in FIG. 3.

The social issue information collection unit 121 may collect social issue information through an Internet communication network and may select and collect social issue information associated with pre-stored genetic markers and phenotype information.

For example, if the fine dust concentration is high and becomes an issue, the information may be collected through newspaper articles or fine dust concentration index data. In general, it has been known that fine dust is associated with asthma. In other words, it has been known that fine dust aggravates the symptoms of asthma patients and may cause asthma when exposed to healthy people for a long time. Genetic markers found to be associated with asthma and the association between fine dust and asthma may be collected by judging and selecting social issue information associated with the genetic marker and phenotype information using an own database.

The genetic test result information/selection storage unit 122 may extract and store social issue-associated genetic test result information that is associated with the social issue information collected through the social issue information collection unit 121 among the genetic test result information. In other words, information associated with the genetic test result information about the user is extracted from the general social issue information related to the previously stored genetic information (genetic markers and phenotypes, etc.).

The user search and activity record information storage unit 123 may interwork with the user's communication device to collect and store and manage the Internet search and activity record information including at least one of the user's Internet search records, video view records, online shopping records, and messenger conversation content.

For example, when the user has recently searched for cosmetics on a PC or mobile device, the corresponding activity records may be collected and stored, and the skin-related phenotypes associated with cosmetics may be judged and selected through an algorithm using an own database. The above-described skin-related phenotypes include skin antioxidability, skin elasticity, skin dryness, pigmentation, and the like.

The personal genetic result and customized information providing unit 130 may provide each of social issue-associated genetic test result information and personalized solution information, related to social issue information and according to the genetic test result information, according to a subscription service cycle and social issue information update time selected by the user, and may extract the social issue and personal interest-associated genetic information based on the user's personal interest based on search and activity record information from the social issue-associated genetic test result information and additionally provide it according to the subscription service cycle, thereby recommending and providing the individual's genetic test result and content information optimized for the individual and having high mutual similarity based on the individual's genetic test result.

To this end, the personal genetic result and customized information providing unit 130 may include a first information providing unit 131, a second information providing unit 132, and a third information providing unit 133 as shown in FIG. 4.

The first information providing unit 131 may provide the user with the social issue-associated genetic test result information and personalized solution information related to the social issue information and according to the genetic test result information, in the form of a periodical, according to the subscription service cycle.

For example, if a high fine dust concentration becomes an issue through newspaper articles, news, or fine dust concentration index data, the first information providing unit 131 may provide information, such as time and place to avoid going out, items to be prepared when going out (such as a mask), and a guide to purchasing the items to the user with a genetic marker found to be related to the occurrence of asthma and the association between fine dust and asthma and may provide the so-provided genetic test result information and personalized solution information in the form of an online/physical periodical, according to the subscription service cycle set by the user.

As such, the first information providing unit 131 provides the genetic test results for disease and phenotypes, in the form of a weekly or monthly, through an app and web, periodically according to the subscription service cycle (e.g., once a month, twice a month, once a week, or once a quarter) selected by the user and selects the disease and phenotypes from its own database and provides the test results including the items associated with the social issues within a recent specific period.

The second information providing unit 132 may provide the user with the social issue-associated genetic test result information and personalized solution information related to the social issue information and according to the genetic test result information, in the form of a push notification popup, each time when the social issue information is updated.

For example, if a high fine dust concentration becomes an issue through newspaper articles, news, or fine dust concentration index data, the second information providing unit 132 may provide information, such as time and place to avoid going out, items to be prepared when going out (such as a mask), and a guide to purchasing the items to the user with a genetic marker found to be related to the occurrence of asthma and the association between fine dust and asthma and may provide the so-provided genetic test result information and personalized solution information in the form of a push popup notification immediately at the time when the corresponding social issue information is updated on the user's portable communication device. Further, the second information providing unit 132 may provide test results, including items associated with social issues within a recent specific period and items highly related to personal interests.

The third information providing unit 133 extracts social issue and personal interest-related genetic information based on the user's personal interest based on search and activity record information from the social issue-associated genetic test result information and provide it to the user according to the subscription service cycle.

For example, when the customer searches for cosmetics, the third information providing unit 133 may provide skin-related genetic test results that are related to the corresponding activity record and may provide various social media (various advertisements, search results, YouTube video recommendations, shopping information) suitable for the individual, as customized recommendation information, along with the genetic test results, using an artificial intelligence algorithm. This personalized content recommendation scheme may be provided in such a way that per-category GUI icons are displayed to be selected by the individual through an application provided separately according to the embodiment, rather than a web browser on a PC or mobile device as conventional.

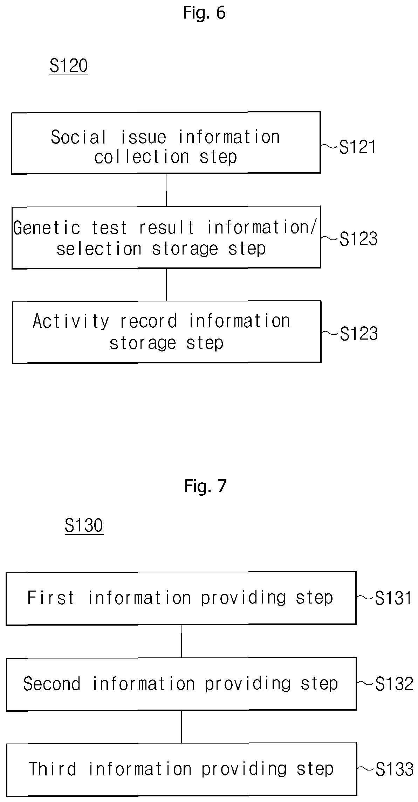

FIG. 5 is a flowchart illustrating a configuration of a genetic information personalized social content information providing method according to another embodiment of the present invention. FIG. 6 is a flowchart illustrating a configuration of an information collection/storage management step according to another embodiment of the present invention. FIG. 7 is a flowchart illustrating a personal genetic result and customized information providing step according to another embodiment of the present invention.

Referring to FIG. 5, according to an embodiment of the present invention, a genetic information personalized social content information providing method S100 includes a genetic test step S110, an information collection/storage management step S120, and a personal genetic result and customized information providing step S130.

The genetic test step S110 may conduct and analyze a genetic test based on a sample collected from the user and provide genetic test result information. For the genetic test, a sample, such as the user's saliva and oral epithelial cells, may be collected, and various genetic tests for genetic mutations, diseases, phenotypes, etc. may be performed on the collected sample, and the test results may be provided.

The genetic test result information (or genome analysis result data) according to the embodiment may be separated (anonymized) from personal identification information, encrypted with a separate unique number, and stored, and be protected through a server security device to prevent the data from leakage. When the user cancels the genome magazine subscription service according to the embodiment or applies for information withdrawal, the customer's genomic data and its derivative information all may be automatically deleted not to be recovered.

Further, the genetic test step S110 may perform and analyze an acquired genetic test based on the sample collected from the user and additionally include epigenetic information related to the genetic marker and phenotype in the genetic test result information and provide it.

More specifically, it is possible to provide more accurate genetic test results by linking and adding epigenetic information, which is acquired genetic information, with the genetic marker and phenotype according to the user's selection. In general, innate genetic information means such information as methyl group (ch3), but since such a type has the same mechanism as all other epigenetic types, such as acetyl group. Thus, fusion of innate genetic information and acquired epigenetic information is not difficult. Further, it is meaningful to provide more accurate information about the user's genes and phenotypic traits using common-sense and universal algorithms.

Further, even when the user's innate genetic information is specified, the acquired epigenetic genetic information may be enhanced through management of lifestyle, aging, and disease and may thus provide the effect of enhancing the quality of life in an acquired manner.

The genetic test step S110 may provide genetic test result information by predicting and analyzing the risk of occurrence of the user's disease and non-disease phenotypes by using the genetic marker, but when updated with a new research result according to a new genetic marker, update the genetic test result information to which the new genetic marker has been applied and provide it. When the existing genetic test result item is updated with the new research result, the genetic test step S 110 adds the corresponding genetic marker information to its own database and collects and stores it.

As such, when the existing genetic test result item is updated with a new research result, the test result update providing method according to the new research result by the genetic test step S110 may apply the new research result thereto to additionally provide a more accurate test result. The genetic test result analysis may be performed in a method of predicting the likelihood (risk) of the occurrence of disease and non-disease phenotypes of the testee by way of the genetic markers reported through existing research. However, the results of genetic research are continuously being published. When a new genetic marker is discovered and new research results are applied, the test results (risk level) may change, so that the test results need to be updated accordingly.

The information collection/storage management step S120 may collect social issue information through an Internet communication network, extract and store social issue-associated genetic test result information, associated with the social issue information, among the genetic test result information and interwork with the user's communication device to collect and store the user's Internet search and activity record information.

To this end, the information collection/storage management step S120 may include a social issue information collection step S121, a genetic test result information/selection storage step S122, and a user search and activity record information storage step S123 as shown in FIG. 6.

The social issue information collection step S121 may collect social issue information through an Internet communication network and may select and collect social issue information associated with pre-stored genetic markers and phenotype information.

For example, if the fine dust concentration is high and becomes an issue, the information may be collected through newspaper articles or fine dust concentration index data. In general, it has been known that fine dust is associated with asthma. In other words, it has been known that fine dust aggravates the symptoms of asthma patients and may cause asthma when exposed to healthy people for a long time. Genetic markers found to be associated with asthma and the association between fine dust and asthma may be collected by judging and selecting social issue information associated with the genetic marker and phenotype information using an own database.

The genetic test result information/selection storage step S122 may extract and store social issue-associated genetic test result information that is associated with the social issue information collected through the social issue information collection step S121 among the genetic test result information. In other words, information associated with the genetic test result information about the user is extracted from the general social issue information related to the previously stored genetic information (genetic markers and phenotypes, etc.).

The user search and activity record information storage step S123 may interwork with the user's communication device to collect and store and manage the Internet search and activity record information including at least one of the user's Internet search records, video view records, online shopping records, and messenger conversation content.

For example, when the user has recently searched for cosmetics on a PC or mobile device, the corresponding activity records may be collected and stored, and the skin-related phenotypes associated with cosmetics may be judged and selected through an algorithm using an own database. The above-described skin-related phenotypes include skin antioxidability, skin elasticity, skin dryness, pigmentation, and the like.

The personal genetic result and customized information providing step S130 may provide each of social issue-associated genetic test result information and personalized solution information, related to social issue information and according to the genetic test result information, according to a subscription service cycle and social issue information update time selected by the user, and may extract the social issue and personal interest-associated genetic information based on the user's personal interest based on search and activity record information from the social issue-associated genetic test result information and additionally provide it according to the subscription service cycle, thereby recommending and providing the individual's genetic test result and content information optimized for the individual and having high mutual similarity based on the individual's genetic test result.

To this end, the personal genetic result and customized information providing step S130 may include a first information providing step S131, a second information providing step S132, and a third information providing step S133 as shown in FIG. 7.

The first information providing step S131 may provide the user with the social issue-associated genetic test result information and personalized solution information related to the social issue information and according to the genetic test result information, in the form of a periodical, according to the subscription service cycle.

For example, if a high fine dust concentration becomes an issue through newspaper articles, news, or fine dust concentration index data, the first information providing step S131 may provide information, such as time and place to avoid going out, items to be prepared when going out (such as a mask), and a guide to purchasing the items to the user with a genetic marker found to be related to the occurrence of asthma and the association between fine dust and asthma and may provide the so-provided genetic test result information and personalized solution information in the form of an online/physical periodical, according to the subscription service cycle set by the user.

As such, the first information providing step S131 provides the genetic test results for disease and phenotypes, in the form of a weekly or monthly, through an app and web, periodically according to the subscription service cycle (e.g., once a month, twice a month, once a week, or once a quarter) selected by the user and selects the disease and phenotypes from its own database and provides the test results including the items associated with the social issues within a recent specific period.

The second information providing step S132 may provide the user with the social issue-associated genetic test result information and personalized solution information related to the social issue information and according to the genetic test result information, in the form of a push notification popup, each time when the social issue information is updated.

For example, if a high fine dust concentration becomes an issue through newspaper articles, news, or fine dust concentration index data, the second information providing step S132 may provide information, such as time and place to avoid going out, items to be prepared when going out (such as a mask), and a guide to purchasing the items to the user with a genetic marker found to be related to the occurrence of asthma and the association between fine dust and asthma and may provide the so-provided genetic test result information and personalized solution information in the form of a push popup notification immediately at the time when the corresponding social issue information is updated on the user's portable communication device. Further, the second information providing step S132 may provide test results, including items associated with social issues within a recent specific period and items highly related to personal interests.

The third information providing step S133 extracts social issue and personal interest-related genetic information based on the user's personal interest based on search and activity record information from the social issue-associated genetic test result information and provide it to the user according to the subscription service cycle.

For example, when the customer searches for cosmetics, the third information providing step S133 may provide skin-related genetic test results that are related to the corresponding activity record and may provide various social media (various advertisements, search results, YouTube video recommendations, shopping information) suitable for the individual, as customized recommendation information, along with the genetic test results, using an artificial intelligence algorithm. This personalized content recommendation scheme may be provided in such a way that per-category GUI icons are displayed to be selected by the individual through an application provided separately according to the embodiment, rather than a web browser on a PC or mobile device as conventional.

According to the embodiment, it is possible to select a genetic test item according to social interest, issue, or individual interest by collecting a sample of the user's blood or saliva and periodically update and provide the report in the form of a periodical.

Further, in the gene report, personalized social media (content) recommendation information according to the genetic test result may also be provided periodically. The information provided is not limited to the genetic information based on the DNA sequence information determined at the time of birth, but the acquired epigenetic information determined by age or environment may be fused and provided. The user who receives the report, i.e., the genome magazine, may periodically analyze and examine the epigenetic genetic information about the sample, obtaining a prediction and related customized social content recommendation information that combines innate genetic information and acquired genetic change information.

## Claims

1. A genetic information personalized social content information providing system, comprising:
a genetic test device unit performing a genetic test and analysis based on a sample collected from a user to provide genetic test result information;
an information collection/storage management unit collecting social issue information through an Internet communication network, extracting social issue-associated genetic test result information associated with the social issue information from the genetic test result information and storing the extracted information, interworking with the user's communication device to collect, store, and manage the user's Internet search and activity record information; and
a personal genetic result and customized information providing unit providing each of the social issue-associated genetic test result information and personalized solution information related to the social issue information and according to the genetic test result information, according to at least one of a subscription service cycle selected by the user and an update time of the social issue information, wherein the personal genetic result and customized information providing unit extracts social issue and personal interest-associated genetic information based on the user's personal interest based on the search and activity record information from the social issue-associated genetic test result information and additionally provides the extracted information according to the subscription service cycle.

2. The genetic information personalized social content information providing system of claim 1, wherein the genetic test device unit performs an acquired genetic test and analysis based on the sample collected from the user to add acquired epigenetic information related to a genetic marker and phenotype to the genetic test result information.

3. The genetic information personalized social content information providing system of claim 1, wherein the genetic test device unit predicts and analyzes the user's risk of occurrence of disease and non-disease phenotype using a genetic marker to provide the genetic test result information and, when updated with a new research result according to a new genetic marker, updates and provides the genetic test result information by applying the new genetic marker.

4. The genetic information personalized social content information providing system of claim 1, wherein the information collection/storage management unit includes:
a social issue information collection unit selecting and collecting the social issue information associated with pre-stored genetic marker and phenotype information through the Internet communication network;
a genetic test result information selection/storage unit extracting the social issue-associated genetic test result information associated with the social issue information collected through the social issue information collection unit from the genetic test result information and storing the extracted information; and
a user search and activity record information storage unit interworking with the user's communication device to collect, store, and manage Internet search and activity record information including at least one of the user's Internet search record, video view record, online shopping record, and messenger conversation.

5. The genetic information personalized social content information providing system of claim 1, wherein the personal genetic result and customized information providing unit includes:
a first information providing unit providing the user with the social issue-associated genetic test result information and the personalized solution information related to the social issue information and according to the genetic test result information, in a form of a periodical, according to the subscription service cycle;
a second information providing unit providing the user with the social issue-associated genetic test result information and the personalized solution information related to the social issue information and according to the genetic test result information, in a form of a push notification popup, each time when the social issue information is updated; and
a third information providing unit extracting the social issue and personal interest-associated genetic information based on the user's personal interest based on the search and activity record information from the social issue-associated genetic test result information and providing the user with the extracted information according to the subscription service cycle.

6. A genetic information personalized social content information providing method, comprising:
a genetic test step performing a genetic test and analysis based on a sample collected from a user to provide genetic test result information, by a genetic test device unit;
an information collection/storage management step collecting social issue information through an Internet communication network, extracting social issue-associated genetic test result information associated with the social issue information from the genetic test result information and storing the extracted information, interworking with the user's communication device to collect, store, and manage the user's Internet search and activity record information, by an information collection/storage management unit; and
a personal genetic result and customized information providing step providing each of the social issue-associated genetic test result information and personalized solution information related to the social issue information and according to the genetic test result information, according to at least one of a subscription service cycle selected by the user and an update time of the social issue information, by a personal genetic result and customized information providing unit, wherein the personal genetic result and customized information providing step extracts social issue and personal interest-associated genetic information based on the user's personal interest based on the search and activity record information from the social issue-associated genetic test result information and additionally provides the extracted information according to the subscription service cycle.

7. The genetic information personalized social content information providing method of claim 6, wherein the genetic test device step performs an acquired genetic test and analysis based on the sample collected from the user to add acquired epigenetic information related to a genetic marker and phenotype to the genetic test result information.

8. The genetic information personalized social content information providing method of claim 6, wherein the genetic test device step predicts and analyzes the user's risk of occurrence of disease and non-disease phenotype using a genetic marker to provide the genetic test result information and, when updated with a new research result according to a new genetic marker, updates and provides the genetic test result information by applying the new genetic marker.

9. The genetic information personalized social content information providing method of claim 6, wherein the information collection/storage management step includes:
a social issue information collection step selecting and collecting the social issue information associated with pre-stored genetic marker and phenotype information through the Internet communication network;
a genetic test result information selection/storage step extracting the social issue-associated genetic test result information associated with the social issue information collected through the social issue information collection step from the genetic test result information and storing the extracted information; and
a user search and activity record information storage step interworking with the user's communication device to collect, store, and manage Internet search and activity record information including at least one of the user's Internet search record, video view record, online shopping record, and messenger conversation.

10. The genetic information personalized social content information providing method of claim 6, wherein the personal genetic result and customized information providing step includes:
a first information providing step providing the user with the social issue-associated genetic test result information and the personalized solution information related to the social issue information and according to the genetic test result information, in a form of a periodical, according to the subscription service cycle;
a second information providing step providing the user with the social issue-associated genetic test result information and the personalized solution information related to the social issue information and according to the genetic test result information, in a form of a push notification popup, each time when the social issue information is updated; and
a third information providing step extracting the social issue and personal interest-associated genetic information based on the user's personal interest based on the search and activity record information from the social issue-associated genetic test result information and providing the user with the extracted information according to the subscription service cycle.
